(19) Europäisches Patentamt
European Patent Office
Office européen des brevets

(11) Veröffentlichungsnummer: **0 206 293 B1**

(12) # EUROPÄISCHE PATENTSCHRIFT

(45) Veröffentlichungstag der Patentschrift: **20.03.91**

(51) Int. Cl.[5]: **C07D 213/64**

(21) Anmeldenummer: **86108475.4**

(22) Anmeldetag: **20.06.86**

(54) **Verfahren zur Herstellung von 3,5-Dichlor-2-pyridon.**

(30) Priorität: **25.06.85 CH 2691/85**

(43) Veröffentlichungstag der Anmeldung:
**30.12.86 Patentblatt 86/52**

(45) Bekanntmachung des Hinweises auf die Patenterteilung:
**20.03.91 Patentblatt 91/12**

(84) Benannte Vertragsstaaten:
**BE CH DE FR GB IT LI LU NL**

(56) Entgegenhaltungen:
**DE-A- 2 141 449**
**DE-C- 597 974**

**J. Org. Chem. 23, 1614(1958)**

(73) Patentinhaber: **LONZA AG**

**Gampel/Wallis(CH)**

(72) Erfinder: **Quarroz, Daniel, Dr.**
**Balfrinstrasse 21**
**Visp (Kanton Wallis)(CH)**

(74) Vertreter: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**W-8000 München 40(DE)**

**EP 0 206 293 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von 3,5-Dichlor-2-pyridon, das seinerseits leicht zum 2,3,5-Trichlorpyridin umgesetzt werden kann.

2,3,5-Trichlorpyridin bildet ein gesuchtes Edukt für die Herstellung einer grossen Palette von Insektiziden [P.Sutter et al, J.Het.Chem.17 , 493 (1980)].

Es ist bekannt, ausgehend von 2-Pyridon durch Chlorierung das 3,5-Dichlor-2-pyridon mit einer Ausbeute von 63% herzustellen [Cava et al, J.Org.Chem.23 , 1614 (1958)].
Gemäss USSR-Patent 194 823 (Appl. 20.4.1966) ist es auch bekannt, ausgehend von 2-Pyridon durch Umsetzung mit tert-Butylhypochlorit das 3,5-Dichlor-2-pyridon im Gemisch mit 5-Chlorpyridon zu erhalten.

Diese bekannten Verfahren haben aber den Nachteil, dass vom relativ schwer zugänglichem 2-Pyridon ausgegangen werden muß.

Das Ziel der vorliegenden Erfindung ist es, ein Verfahren aufzuzeigen, das erlaubt, von einem günstigen Edukt ausgehend auf einfache Weise und in guter Ausbeute und Qualität zum gewünschten 3,5-Dichlor-2-pyridon zu gelangen.
Dies wird erreicht nach einem Verfahren gemäss dem Patentanspruch 1, worin erfindungsgemäss 6-Hydroxynikotinsäure mit Chlor oder chlorabgebenden Agenzien umgesetzt wird.

Als geeignete chlorabgebende Agenzien werden die Hypochlorite, besonders die Alkali- oder Erdalkalimetallhypochlorite angesehen. Ganz besonders geeignet ist Natrium- oder Kaliumhypochlorit.
Die Hypochlorite werden zweckmässig gelöst in Wasser in einer Konzentration von 5 bis 25%, vorzugsweise von 8 bis 14%, zugesetzt.
Die Chlorierungsagenzien werden allgemein in einem Ueberschuss, bezogen auf das stöchiometrische Verhältnis, zugesetzt. Dieser Ueberschuss beträgt bei den Hypochloriten zweckmässig 0 bis 300%, vorzugsweise 20 bis 50%.
Wird Chlor verwendet, beträgt der Ueberschuss zweckmässig 0 bis 300%, vorzugsweise 20 bis 50%.

Zweckmässig wird das Verfahren in Gegenwart von Wasser als Lösungsmittel durchgeführt.

Die geeignete Reaktionstemperatur liegt zweckmässig zwischen 0 und 40 °C, vorzugsweise zwischen 0 und 20 °C.

Es wird ausserdem darauf geachtet, dass der pH-Bereich zwischen zweckmässig 7 bis 12, bei Verwendung der Hypochlorite sowie von Chlor als Chlorierungsagens vorzugsweise zwischen 8 bis 11 liegt.

Nach beendeter Halogenierung kann auf einfache Weise, z.B. durch Filtration oder Extraktion, das 3,5-Dichlor-2-pyridon aus der Reaktionslösung entfernt werden.

Die Ausbeuten liegen im allgemeinen über 70%.

Gegebenenfalls kann das Produkt durch Umkristallisation, z.B. in Wasser, Ethylacetat oder Toluol, gereinigt werden. Ueberlicherweise genügt aber die Qualität des Rohproduktes zur Weiterumsetzung, z.B. entsprechend US-PS 4 287 347, zum 2,3,5-Trichlorpyridin.

### Beispiel 1

70 g (0,5 Mol) 6-Hydroxynikotinsäure wurden in 500 ml Wasser aufgeschlämmt und anschliessend mit Natronlauge in Lösung gebracht (pH der Lösung = 10). Bei 0 °C wurden langsam 100 g Chlor eingeleitet. Der pH wurde durch NaOH-Zugabe zwischen 10 und 11 gehalten.
Nach 2 Stunden war die Chlorierung beendet. Bei pH 5 wurde der erhaltene, leicht gelbe Niederschlag abgenutscht, mit Wasser gewaschen und anschliessend unter Vakuum bei 50 °C getrocknet.
Man erhielt 63,5 g = 77,4% 3,5-Dichlor-2-pyridon.
$^1$H-NMR ($CDCl_3$): δ(ppm); 7,5 (d); 7,72 (d).

### Beispiel 2

8,3 g (0,06 Mol) 6-Hydroxynikotinsäure wurden in 30 ml Wasser aufgeschlämmt und anschliessend mit Natronlauge in Lösung gebracht (pH der Lösung = 8). Bei 5 °C gab man langsam 76,5 g einer 13,6%igen Lösung von NaOCl in Wasser zu. Während der Zugabe wurde der pH zwischen 7 und 8,5 gehalten.
Nach beendeter Reaktion wurde der entstandene, leicht gelbe Niederschlag abgenutscht, mit Wasser gewaschen und anschliessend unter Vakuum bei 50 °C getrocknet.
Man erhielt 7,0 g = 71,1% 3,5-Dichlor-2-pyridon.
$^1$H-NMR ($CDCl_3$): δ (ppm); 7,5 (d); 7,72 (d).

## Ansprüche

1. Verfahren zur Herstellung von 3,5-Dichlor-2-pyridon der Formel

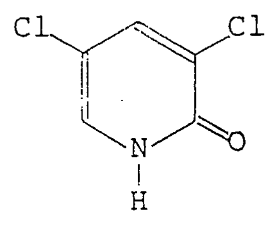

dadurch gekennzeichnet, dass 6-Hydroxynikotinsäure mit Chlor oder einem chlorabgebenden Agens umgesetzt wird.

2. Verfahren nach Patentanspruch 1, dadurch gekennzeichnet, dass als chlorabgebendes Agens die Alkali- oder Erdalkalimetallhypochlorite verwendet werden.

3. Verfahren nach Patentanspruch 2, dadurch gekennzeichnet, dass als chlorabgebendes Agens Natrium- oder Kaliumhypochlorit verwendet wird.

4. Verfahren nach Patentansprüchen 1 bis 3, dadurch gekennzeichnet, dass in Gegenwart von Wasser als Lösungsmittel gearbeitet wird.

5. Verfahren nach Patentansprüchen 1 bis 4, dadurch gekennzeichnet, dass die Reaktion in einem pH-Bereich von 7 bis 12 durchgeführt wird.

6. Verfahren nach Patentansprüchen 1 bis 5, dadurch gekennzeichnet, dass bei Temperaturen von 0 bis +40 °C gearbeitet wird.

## Claims

1. Process for the preparation of 3,5-dichloro-2-pyridone of the formula

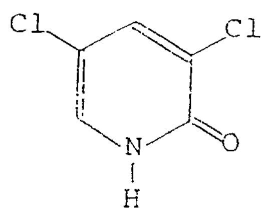

characterized in that 6-hydroxynicotinic acid is reacted with chlorine or a chlorine-releasing agent.

2. Process according to patent claim 1, characterized in that as chlorine-releasing agents alkali metal or alkaline earth metal hypochlorites are used.

3. Process according to patent claim 2, characterized in that as chlorine-releasing agent sodium or potassium hypochlorite is used.

4. Process according to patent claims 1 to 3, characterized in that one operates in the presence of water as solvent.

5. Process according to patent claims 1 to 4, characterized in that the reaction is carried out in a pH-range of 7 to 12.

6. Process according to patent claims 1 to 5, characterized in that one operates at temperatures of 0 to +40 °C.

## Revendications

1. Procédé de préparation de la 3,5-dichloro-2-pyridone de formule

Figure 1

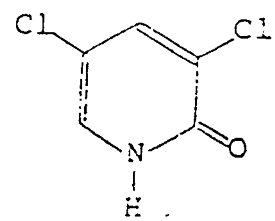

caractérisé en ce que l'on fait réagir l'acide 6-hyroxynicotinique avec du chlore ou un agent cédant du chlore.

2. Procédé selon la revendication 1, caractérisé en ce que l'on utilise comme agent cédant du chlore, les hypochlorites de métaux alcalins ou alcalino-terreux.

3. Procédé selon la revendication 2, caractérisé en ce que l'on utilise comme agent cédant du chlore l'hypochlorite de sodium ou de potassium.

4. Procédé selon les revendications 1 à 3, caractérisé en ce que l'on travaille en présence d'eau comme solvant.

5. Procédé selon les revendications 1 à 4, caractérisé en ce que la réaction est effectuée dans un domaine de pH de 7 à 12.

6. Procédé selon les revendications 1 à 5, caractérisé en ce que l'on travaille à des températures de 0 à +40 °C.